# EUROPÄISCHE PATENTSCHRIFT

(11) **EP 0 609 796 B1**
(45) Veröffentlichungstag und Bekanntmachung des Hinweises auf die Patenterteilung: **02.12.1998**
(21) Anmeldenummer: 94101350.0
(22) Anmeldetag: 29.01.1994
(51) Int. Cl.: A61K 7/135

(54) **Blondiermittel für menschliche Haare**
Hairbleaching composition
Composition pour décoloré des cheveux humains

(30) Priorität: 01.02.1993 DE 4302738; 31.08.1993 DE 4329282
(43) Veröffentlichungstag der Anmeldung: 10.08.1994
(73) Patentinhaber: GOLDWELL GmbH, D-64297 Darmstadt (DE)
(72) Erfinder: Lorenz, Heribert, D-64401 Gross-Bieberau (DE); Eberling, Walter, D-64560 Riedstadt-Crumstadt (DE); Simon, Frederic, D-64569 Nauheim (DE)

(56) Entgegenhaltungen:
- DE-A- 1 617 829
- DE-A- 2 023 922
- DE-A- 2 307 596
- DE-A- 3 814 356
- FR-A- 1 257 661
- GB-A- 2 242 358
- NL-C- 78 053

## Beschreibung

Die vorliegende Erfindung betrifft ein Wasserfreies Blondiermittel für menschliche Haare, vorzugsweise in Pulverform, das insbesondere wesentlich verbesserte physiologische Eigenschaften aufweist.

Blondierpulver sind seit langem bekannt und im Einsatz. Sie enthalten in aller Regel eine pulverförmige Grundlage mit verschiedenen Zusätzen, insbesondere Alkalisierungsmitteln, um den pH-Wert bei der Anwendung bei etwa 9,5 bis 10,5 einzustellen.

Als Aktivsubstanz ist ein Peroxid, in der Regel ein Persulfat, insbesondere Ammonium-, Natrium- oder Kaliumpersulfat, enthalten. Diese Pulver werden bei der Anwendung mit wäßriger, vorzugsweise 6%iger Wasserstoffperoxid-Lösung vermischt (vgl. hierzu, beispielsweise, die DE-A 1617829).

Es hat sich jedoch gezeigt, daß die Verwendung von Peroxiden, insbesondere Persulfaten, bei bestimmten Personen zu Allergien führen kann. Es wurde deshalb bereits versucht, persulfatfreie Blondiermittel zu verwenden und durch Erhöhung der Wasserstoffperoxidmenge die gleiche Konzentration an reagiblem Sauerstoff zur Verfügung zu stellen. Dabei wurde jedoch überraschenderweise festgestellt, daß durch diese Maßnahme eine qualitativ gleichwertige Aufhellung dem Haares, wie sie mit persulfathaltigen Produkten in Kombination mit Wasserstoffperoxid erhalten wird; mit persulfatfreien Produkten nicht erzielt werden kann.

Es bestand daher die Aufgabe, ein Blondierpulver zu schaffen, das in Kombination mit Wasserstoffperoxid voll wirksam ist, jedoch die mit konventionellen, peroxid-, insbesondere persulfathaltigen Produkten beobachteten physiologischen Probleme nicht aufweist.

Es wurde nunmehr gefunden, daß es möglich ist, ein Blondiermittel, insbesondere ein Blondierpulver herzustellen, das völlig peroxidfrei ist, und trotzdem, in Kombination mit Wasserstoffperoxid, eine ausgezeichnete Blondierwirkung ergibt, wenn man das Peroxid, d.h. das Persulfat, durch mindestens ein Ammoniumsalz, insbesondere Ammoniumcarbonat, Ammoniumhydrogencarbonat und/oder Ammoniumcarbamat, ersetzt und die Zusammensetzung so einstellt, daß beim Mischen mit wäßriger Wasserstoffperoxid-Lösung ein pH-Wert der gebrauchsfertigen Mischung von etwa 7,7 bis etwa 9,0 erhalten wird.

Gegenstand der vorliegenden Erfindung ist daher ein vorzugsweise wasserfreies, insbesondere pulverförmiges Mittel zum Blondieren von menschlichen Haaren, das, bezogen auf die Gesamtzusammensetzung, etwa 10 bis 60, vorzugsweise 15 bis 50, insbesondere 20 bis 40 Gew.-% mindestens eines Ammoniumsalzes, insbesondere Ammoniumcarbonat, Ammoniumhydrogencarbonat und/oder Ammoniumcarbamat, enthält, peroxidfrei und so eingestellt ist, daß beim Vermischen mit Wasserstoffperoxid-Lösung ein pH-Wert zwischen etwa 7,7 und etwa 9,0, vorzugsweise zwischen etwa 8,3 und 8,8, insbesondere etwa 8,6 und 8,7, erreicht wird.

Es hat sich nämlich gezeigt, daß bei der Einstellung eines solchen pH-Wertes eine optimale Blondierwirkung erzielt werden kann.

Die Verwendung von Ammoniumhydrogencarbonat und Ammoniumcarbonat als Alkalisierungsmittel in geringen Mengen in Blondiermitteln ist an sich bereits bekannt (vgl. K.Schrader, "Grundlagen und Rezepturen der Kosmetika", 2. Auflage (1989), S. 815 bis 819), jedoch läßt sich daraus selbstverständlich nicht ableiten, daß durch Ammoniumsalze ein Ersatz des Persulfats bei Beibehaltung der aufhellenden Eigenschaften möglich sein würde.

Neben dem bevorzugt als Ammoniumsalze verwendeten Ammoniumcarbonat, Ammoniumhydrogencarbonat bzw. Ammoniumcarbamat können auch Ammoniumchlorid, Ammoniumsulfat, Ammoniumphosphate, Ammoniumnitrat, Ammoniumbromid, Ammoniumjodid, Ammoniumthiosulfat, Ammoniummolybdat, Ammoniumvanadat, Ammoniumsulfamat, Ammoniumcitrat, Ammoniumsalicylat, Ammoniumvalerat, Ammoniumtartrat, Ammoniumbenzoat, Ammoniumacetat, Ammoniumformiat und Ammoniumlactat verwendet werden.

Bevorzugt sind hierbei die Ammoniumphosphate wie NH₄H₂PO₄, (NH₄)₂HPO₄, (NH₄)₂NaPO₄, NaNH₄HPO₄ oder NH₄Na₂PO₄, Ammoniumchlorid, Ammoniumsulfat und Diammoniumhydrogencitrat.

Neben den Ammoniumsalzen enthalten die erfindungsgemäßen Blondiermittel mit verringertem allergenen Potential die in solchen Mitteln üblichen Bestandteile, insbesondere, wenn es sich um ein Pulver handelt, inerte pulverförmige Trägerstoffe, beispielsweise pyrogenes Siliciumdioxid, und Stärkepulver, Alkalisierungsmittel wie Natriummetasilikat, oberflächenaktive Substanzen, und Bindemittel; zur Vermeidung von Wiederholungen wird auf die einschlägigen Standardwerke, beispielsweise K.Schrader, "Grundlagen und Rezepturen der Kosmetika", 2. Auflage (1989, Hüthig Buchverlag), S.815 bis 823, verwiesen.

Diese Pulver können auch, wie aus der Patentanmeldung P 42 07 475.4 bekannt, mit Öl bzw. mit einem flüssigen Wachs beschichtet sein oder, wie es in der deutschen Patentanmeldung P 42 17 120.3 beschrieben ist, als mit Binde- und Verdickungsmitteln verfestigtes Agglomerat vorliegen.

Obwohl die bevorzugte Ausführungsform des erfindungsgemäßen Mittels ein wasserfreies Blondierpulver darstellt, ist es auch möglich, andere Trägermaterialien zu verwenden, beispielsweise wasserfreie Suspensionen oder Pasten, wie sie z.B. in der DE-A 38 14 356 beschrieben sind.

Die folgenden Beispiele illustrieren die Erfindung.

Es wird eine Grundrezeptur A für ein Blondierpulver hergestellt, die in den Beispielen A-1 bis A-9 als Grundlage verwendet wird:

| Zusammensetzung A | |
|---|---|
| Kieselgur | 3,0 (Gew.-%) |
| Pyrogenes Silica | 8,5 |
| Natriummetasilikat | 35,0 |
| Stärkepulver | 50,0 |
| Eiweißhydrolysat | 0,5 |
| Natriumstearat | 1,5 |
| Natriumcocoyltaurat | 1,0 |
| Natriumcarboxymethylcellulose | 0,5 |

Mit der Grundrezeptur A wurden jeweils die Beispiele A-1 bis A-9 hergestellt, wobei jeweils Citronensäure in den angegebenen Mengen zugesetzt wurde, um im gebrauchsfertigen Produkt, d.h. nach dem Vermischen mit wäßriger Wasserstoffperoxid-Lösung, einen pH-Wert von 8,5 bis 8,7 zu erreichen.

| Beispiel A-1 | |
|---|---|
| Grundrezeptur | 11,6 g |
| Ammoniumhydrogencarbonat | 8,0 g |
| Citronensäure | 0,4 g |

| Beispiel A-2 | |
|---|---|
| Grundrezeptur | 5,6 g |
| Ammoniumcarbamat | 8,0 g |
| Citronensäure | 6,4 g |

| Beispiel A-3 | |
|---|---|
| Grundrezeptur | 12,0 g |
| Ammoniumhydrogencarbonat | 4,0 g |
| Citronensäure | 4,0 g |

| Beispiel A-4 | |
|---|---|
| Grundrezeptur | 10,0 g |
| Ammoniumcarbamat | 4,0 g |
| Citronensäure | 6,0 g |

| Beispiel A-5 | |
|---|---|
| Grundrezeptur | 13,0 g |
| Ammoniumhydrogencarbonat | 2,0 g |
| Citronensäure | 5,0 g |

| Beispiel A-6 | |
|---|---|
| Grundrezeptur | 12,4 g |
| Ammoniumcarbamat | 2,0 g |
| Citronensäure | 5,6 g |

| Beispiel A-7 (nicht erfindungsgemäß) | |
|---|---|
| Grundrezeptur | 13,6 g |
| Ammoniumpersulfat | 1,0 g |
| Kaliumpersulfat | 0,8 g |
| Citronensäure | 4,6 g |

| Beispiel A-8 (nicht erfindungsgemäß) | |
|---|---|
| Grundrezeptur | 8,8 g |
| Ammoniumhydrogencarbonat | 9,0 g |
| Ammoniumpersulfat | 1,0 g |
| Kaliumpersulfat | 0,8 g |
| Natriummetasilikat | 0,4 g |
| (zur pH-Wert-Einstellung auf 8,7) | |

| Beispiel A-9 | |
|---|---|
| Grundrezeptur | 7,6 g |
| Ammoniumcarbonat | 8,6 g |
| Citronensäure | 4,4 g |

Jeweils 20 g der Grundrezeptur A und der Zusammensetzungen nach den Beispielen A-1 bis A-9 wurden mit jeweils 40 ml 6prozentiger Wasserstoffperoxid-Lösung vermischt und mit der erhaltenen Mischung (pH 8,5 - 8,7) Haarsträhnen bei 40 °C 30 Minuten blondiert.

Anschließend wurden die Strähnen gewaschen, getrocknet und der Grad der erzielten Aufhellung verglichen. Die Ergebnisse sind in der folgenden Tabelle wiedergegeben:

| | | | | | | |
|---|---|---|---|---|---|---|
| Beispiel: | A | A-1 | A-2 | A-3 | A-4 | A-5 |
| Aufhellungsgrad: | Keiner | Stark | Stark | Gut | Gut | Sichtbar |

| | | | | |
|---|---|---|---|---|
| Beispiel: | A-6 | A-7 | A-8 | A-9 |
| Aufhellungsgrad: | Sichtbar | Schwach | Stark | Stark |

Die Resultate dieser Versuche zeigen die aufhellende Wirksamkeit der erfindungsgemäßen Zusammensetzungen auch in Abwesenheit von Persulfat.

Es wird eine weitere Grundrezeptur B für ein Blondierpulver hergestellt, die in den Beispielen B-1 bis B-12 als Grundlage verwendet wird:

| Zusammensetzung B | |
|---|---|
| Diatomeenerde | 30,0 (Gew.-%) |
| Silica | 27,0 |
| Hydroxyethylcellulose | 1,5 |
| Cellulose Gum | 6,0 |
| Eiweißhydrolysat | 5,5 |
| Natriumstearat | 16,0 |
| Natriumlaurylsulfat | 2,0 |
| Natriumlauroyltaurat | 6,0 |
| Harnstoff | 4,0 |
| Komplexbildner (EDTA) | 2,0 |

| Beispiel B-1 | |
|---|---|
| Grundrezeptur | 8,0 g |
| Ammoniumchlorid | 8,0 g |
| Natriumcarbonat | 4,0 g |
| (pH: 8,62) | |

| Beispiel B-2 | |
|---|---|
| Grundrezeptur | 12,2 g |
| Ammoniumchlorid | 4,0 g |
| Natriumcarbonat | 3,8 g |
| (pH: 8,70) | |

| Beispiel B-3 | |
|---|---|
| Grundrezeptur | 14,3 g |
| Ammoniumchlorid | 2,0 g |
| Natriumcarbonat | 3,7 g |
| (pH: 8,70) | |

| Beispiel B-4 | |
|---|---|
| Grundrezeptur | 8,0 g |
| Ammoniumsulfat | 8,0 g |
| Natriumcarbonat | 4,0 g |
| (pH: 8,70) | |

| Beispiel B-5 | |
|---|---|
| Grundrezeptur | 12,2 g |
| Ammoniumsulfat | 4,0 g |
| Natriumcarbonat | 3,8 g |
| (pH: 8,70) | |

| Beispiel B-6 | |
|---|---|
| Grundrezeptur | 14,3 g |
| Ammoniumsulfat | 4,0 g |
| Natriumcarbonat | 3,7 g |
| (pH: 8,70) | |

| Beispiel B-7 | |
|---|---|
| Grundrezeptur | 9,4 g |
| Diammoniumhydrogenphosphat | 8,0 g |
| Natriumcarbonat | 2,6 g |
| (pH: 8,70) | |

| Beispiel B-8 | |
|---|---|
| Grundrezeptur | 13,5 g |
| Diammoniumhydrogenphosphat | 4,0 g |
| Natriumcarbonat | 2,5 g |
| (pH: 8,60) | |

| Beispiel B-9 | |
|---|---|
| Grundrezeptur | 16,5 g |
| Diammoniumhydrogenphosphat | 2,0 g |
| Natriumcarbonat | 1,5 g |
| (pH: 8,60) | |

| Beispiel B-10 | |
|---|---|
| Grundrezeptur | 6,6 g |
| Diammoniumhydrogencitrat | 8,0 g |
| Natriumcarbonat | 5,4 g |
| (pH 8,52) | |

| Beispiel B-11 | |
|---|---|
| Grundrezeptur | 10,6 g |
| Diammoniumhydrogencitrat | 4,0 g |
| Natriumcarbonat | 5,4 |
| (pH: 8,80) | |

| Beispiel B-12 | |
|---|---|
| Grundrezeptur | 12,8 g |
| Diammoniumhydrogencitrat | 2,0 g |
| Natriumcarbonat | 5,2 g |
| (pH: 8,80) | |

Jeweils 20 g der Grundrezeptur B und der Zusammensetzungen nach den Beispielen B-1 bis B-12 wurden mit jeweils 40 ml 6%iger Wasserstoffperoxid-Lösung vermischt und mit der erhaltenen Mischung (pH 8,5 - 8,8) Haarsträhnen bei 40 °C 30 Minuten blondiert.

Anschließend wurden die Strähnen gewaschen, getrocknet und der Grad der erzielten Aufhellung verglichen. Die Ergebnisse sind in der folgenden Tabelle wiedergegeben:

| | | | | | |
|---|---|---|---|---|---|
| Beispiel: | B | B-1 | B-2 | B-3 | B-4 |
| Aufhellungsgrad: | Keiner | Stark | Stark | Gut | Stark |

| | | | | |
|---|---|---|---|---|
| Beispiel: | B-5 | B-6 | B-7 | B-8 |
| Aufhellungsgrad: | Gut | Gut | Stark | Gut |

| | | | | |
|---|---|---|---|---|
| Beispiel: | B-9 | B-10 | B-11 | B-12 |
| Aufhellungsgrad: | Gut | Stark | Stark | Gut |

Die Resultate dieser Versuche zeigen die aufhellende Wirksamkeit der erfindungsgemäßen Zusammensetzungen auch in Abwesenheit von Persulfat.

## Patentansprüche

1. Wasserfreies Mittel zum Blondieren von menschlichen Haaren, vorzugsweise in Pulverform, das unmittelbar vor der Anwendung mit wäßriger Wasserstoffperoxidlösung zu einem gebrauchsfertigen Produkt angerührt wird, dadurch gekennzeichnet, daß es 10 bis 60 Gew.-% mindestens eines Ammoniumsalzes, berechnet auf die Gesamtzusammensetzung, enthält sowie peroxidfrei ist und so eingestellt ist, daß beim Mischen des Mittels mit wäßriger Wasserstoffperoxid-Lösung ein pH-Wert der gebrauchsfertigen Mischung zwischen etwa 7,7 und etwa 9,0 erhalten wird.

2. Blondiermittel nach Anspruch 1 , dadurch gekennzeichnet, daß es 20 bis 40 Gew.-% mindestens eines Ammoniumsalzes enthält.

3. Blondiermittel nach Anspruch 1 und/oder 2, dadurch gekennzeichnet, daß es als Ammoniumsalz Ammoniumcarbonat, Ammoniumhydrogencarbonat und/oder Ammoniumcarbamat enthält.

4. Blondiermittel nach Anspruch 1 oder 2, dadurch gekennzeichnet, daß es mindestens ein Ammoniumsalz, ausgewählt aus der Gruppe Ammoniumchlorid, Ammoniumsulfat, Ammoniumphosphate, Ammoniumbromid, Ammoniumjodid, Ammoniumnitrat, Ammoniumthiosulfat, Ammoniummolybdat, Ammoniumvanadat, Ammoniumsulfamat, Ammoniumcitrat, Ammoniumsalicylat, Ammoniumvalerat, Ammoniumtartrat, Ammoniumbenzoat, Ammoniumacetat, Ammoniumformiat und/oder Ammoniumlactat, enthält.

5. Blondiermittel nach Anspruch 4, dadurch gekennzeichnet, daß es mindestens eine Ammoniumverbindung, ausgewählt aus der Gruppe Ammoniumchlorid, Ammoniumsulfat, Ammoniumdihydrogenphosphat, Diammoniummonohydrogenphosphat, Ammoniumnatriumphosphat und Ammoniumcitrat, enthält.

6. Blondiermittel nach einem oder mehreren der Ansprüche 1 bis 5, dadurch gekennzeichnet, daß es zusätzlich Natriumcarbonat enthält.

## Claims

1. Water-free bleaching composition for human hair, preferably in pulverulent form, which is mixed with aqueous hydrogen peroxide solution immediatly before application to obtain a ready-to-use product, characterized in that it contains 10 to 60 % by wt. of at least one ammonium salt, calculated to the total composition, being free from peroxide and being adjusted to reach a pH-value between about 7.7 and about 9.0 of the ready-to-use mixture upon mixing of the composition with aqueous hydrogen peroxide solution.

2. Bleaching composition according to claim 1 characterized in that it contains 20 to 40% by wt. of at least one ammonium salt.

3. Bleaching composition according to claim 1 or 2, characterized in that it contains as ammonium salt ammonium carbonate, ammonium hydrogen carbonate and/or ammonium carbamate.

4. Bleaching composition according to claim 1 or 2 characterized in that it contains at least one ammonium salt selected from the group of ammonium chloride, ammonium sulfate, ammonium phosphate, ammonium bromide, ammonium iodide, ammonium nitrate, ammonium thiosulfate, ammonium molybdate, ammonium vanadate, ammonium sulfamate, ammonium citrate, ammonium salicylate, ammonium valerate, ammonium tartrate, ammonium benzoate, ammonium acetate, ammonium formiate, and ammonium lactate.

5. Bleaching composition according to claim 4, characterized in that it contains at least one ammonium compound selected from the group ammonium chloride, ammonium sulfate, ammonium dihydrogen phosphate, diammonium monohydrogen phosphate, ammonium sodium phosphate and ammonium citrate.

6. Bleaching composition according to one more of the claims 1 to 5, characterized in that it additionally contains sodium carbonate.

## Revendications

1. Agent sans eau pour blondir les cheveux, de préférence sous forme de poudre, à délayer immédiatement avant l'usage avec une solution d'eau oxygénée pour donner un produit prêt-à-l'emploi, caractérisé en ce qu'il contient de 10 à 60% en poids d'au moins un sel d'ammonium par rapport à la composition totale, est exempt de peroxyde et est ajusté de telle manière que l'on obtienne, lors du mélange de l'agent avec la solution d'eau oxygénée, un pH du mélange prêt-à-l'emploi compris entre environ 7,7 et environ 9,0.

2. Agent décolorant selon la revendication 1, caractérisé en ce qu'il contient de 20 à 40% en poids d'au moins un sel d'ammonium.

3. Agent décolorant selon la revendication 1 et/ou 2, caractérisé en ce qu'il contient, comme sel d'ammonium du carbonate, de l'hydrogéno-carbonate d' et/ou du carbamate d'ammonium.

4. Agent décolorant selon la revendication 1 ou 2, caractérisé en ce qu'il contient au moins un sel d'ammonium choisi parmi le groupe constitué du chlorure, du sulfate, du phosphate, du bromure, de l'iodure , du nitrate, du thiosulfate, du molybdate, du vanadate, du sulfamate, du citrate, du salicylate, du valérate, du tartrate, du benzoate, de l'acétate, du formiate et/ou du lactate d'ammonium.

5. Agent décolorant selon la revendication 4, caractérisé en ce qu'il contient au moins un composé ammonium choisi parmi le groupe constitué du chlorure, du sulfate, du dihydrogéno-phosphate d'ammonium, du monohydrogéno-phosphate de diammonium, du phosphate sodique et du citrate d'ammonium.

6. Agent décolorant selon l'une ou plusieurs des revendications 1 à 5, caractérisé en ce qu'il contient en outre du carbonate de sodium.
